# EUROPEAN PATENT SPECIFICATION

(11) **EP 1 198 477 B1**
(45) Date of publication and mention of the grant of the patent: **20.10.2004**
(21) Application number: 00951554.5
(22) Date of filing: 03.08.2000
(51) Int. Cl.: C07K 14/575, C07K 14/72, A61K 38/22, A61P 5/00, A61P 25/04, G01N 33/68

(54) **PROLACTIN-RELEASING PEPTIDE AND USE IN TREATING PAIN**
PROLAKTIN FREISETZENDES PEPTID UND VERWENDUNG ZUR BEHANDLUNG VON SCHMERZEN
PEPTIDE DE LIBERATION DE LA PROLACTINE ET UTILISATION DANS LE TRAITEMENT DE LA DOULEUR

(30) Priority: 03.08.1999 US 365756; 20.03.2000 US 531567
(43) Date of publication of application: 24.04.2002
(73) Proprietor: Oy Juvantia Pharma Ltd, 20520 Turku (FI)
(72) Inventor: PANULA, Pertti, Aarre, Juhani, FIN-01420 Jorvas (FI); PERTOVAARA, Antti, FIN-00420 Helsinki (FI); KALSO, Eija, FIN-02100 Espoo (FI); KORPI, Esa, FIN-02200 Espoo (FI)
(74) Representative: Risku, Ira Marjatta
(86) International application number: PCT/FI2000/000664
(87) International publication number: WO 2001/009182

(56) References cited:
- WO-A1-00/38704
- WO-A1-99/60112
- WO-A2-97/24436
- DATABASE MEDLINE [Online] ROLAND B.L. ET AL.: 'Anatomical distribution of prolactin-releasing peptide and its receptor suggests additional functions in the central nervous system and periphery', XP002935331 Retrieved from Dialog Information Services, File 155, accession no. 10194899 Database accession no. 20043940 & ENDOCRINOLOGY vol. 140, no. 12, December 1999, (UNITED STATES), pages 5736 - 5745
- W.K. SAMSON ET AL.: 'A non-neuroendocrine action of prolactin releasing peptide-20: autonomic control' SOCIETY FOR NEUROSCIENCE vol. 25, no. 1-2, 1999, page 1692, XP002935332
- SHUJI HINUMA ET AL.: 'A prolactin-releasing peptide in the brain' NATURE vol. 393, May 1998, pages 272 - 276, XP002935333
- SHIRO MINAMI ET AL.: 'Cellular iocalization of prolactin-releasing peptide messenger RNA in the rat brain' NEUROSCIENCE LETTERS vol. 266, 1999, pages 73 - 75, XP002935334
- DATABASE EMBASE [Online] AARNISALO A.A. ET AL.: 'Evidence for prolactin releasing activity of neuropeptide FF in rats', XP002935335 Retrieved from Dialog Information Services, File 73, accession no. 07251871 Database accession no. 1998149283 & NEUROENDOCRINOLOGY LETTERS vol. 18, no. 4, 1997, (UNITED KINGDOM), pages 191 - 196
- DATABASE MEDLINE [Online] PANULA P. ET AL.: 'Neuropeptide FF, a mammalian neuropeptide with multiple functions', XP002935336 Retrieved from Dialog Information Services, File 155, accession no. 08856507 Database accession no. 96397012 & PROGRESS IN NEUROBIOLOGY vol. 48, no. 4-5, March 1996 - April 1996, (ENGLAND), pages 461 - 487
- MARUYAMA ET AL: 'Immunocytochemical localization of prolactin-releasing peptide in the rat brain' ENDOCRINOLOGY vol. 140, no. 6, 1999, pages 2326 - 2333

## Description

### FIELD OF THE INVENTION

The present invention relates to the use of prolactin-releasing peptide (PrRP) for preparing a medicament for treating pain. This peptide regulates blood pressure and pain mechanisms, and is expressed in complementary areas with neuropeptide FF (NPFF). Prolactin-releasing peptide and neuropeptide FF are both RF-amide peptides.

### BACKGROUND OF THE INVENTION

The first mammalian RF-amide peptide neuropeptide FF (NPFF) was identified from bovine brain (Yang et al. 1985) and localized to a limited number of neural systems in rat central nervous system (CNS) (Kivipelto et al. 1989, Panula et al. 1996). The rat and bovine gene were found to be highly homologous (Vilim et al. 1999), which suggests that the gene is highly conserved. In addition to the brain, the peptide is found in human plasma (Sundblom et al. 1996), and the human gene (Perry et al. 1997, Vilim et al. 1999) shares the basic structure with that of other mammals. It is known that NPFF has both potent pro-opioid pain relieving effects and antiopioid-like effects, depending on site of administration and dose, and it regulates blood pressure (Panula et al. 1996).

Expression of NPFF gene as studied using in situ hybridization (Vilim et al. 1999), and immunocytochemistry (for review see Panula et al. 1996) display some differences: a central hypothalamic nucleus contains cellular peptide immunoreactivity but does not display NPFF mRNA. These results have been explained by the possibility that several genes may give rise to RF-amide peptides in mammals (Panula et al. 1996, Vilim et al. 1999). Evidence from both in vivo and in vitro studies suggest that NPFF releases prolactin in rats (Aarnisalo et al. 1997). Recently, another mammalian RF-amide peptide was identified as prolactin-releasing peptide (PrRP) and cloned (Hinuma et al. 1998). However, it has been found out that the prolactin-releasing effect of this cloned PrRP is more moderate than Hinuma et al. originally believed. In spite of this, the name of the peptide has, for congruency, not been changed in this application.

Although there exist two short reports on the expression sites of PrRP gene in rat brain using in situ hybridization (Minami et al. 1999) and immunohistochemistry (Chen et al. 1999), the authors did not report the distribution of nerve terminals (sites of action) or the receptor. Furthermore, they did not suggest the involvement of PrRP and its receptor in autonomic regulation and pain. This study is the first one where the sites of action and direct effects of the applied peptide on pain are found.

The G-protein coupled receptor UHR-1 was identified as a receptor of unknown function from the rat brain (Welch et al. 1995). A related human receptor GPR10 was cloned from human tissue (Marchese et al. 1995), and identified as a PrRP receptor coupled to arachidonic acid pathway (Hinuma et al. 1998).

An article of Maruyama et al., Endocrinology vol. 140(6), pp 2326-2333, describes the distribution of prolactin-releasing peptide in rat brain using immunohistochemistry. Most emphasis is given to the hormone-releasing systems of the hypothalamus and pituitary. The paper does not specifically address the spinal and brain stem systems related to pain and no data or reference is included to provide any evidence of effect of PrRP on either acute or neuropathic pain.

WO 0038704 discloses the immunological detection of PrRP protein but use of the protein in pain treatment is not mentioned.

In WO 9960112 antibodies to PrRP20 have been disclosed and used for immunoassay to detect PrRP20. The document does not include any teaching or suggestion about the usefulness for pain treatment of the protein.

WO 9724434 relates to the ligand polypeptide for the human pituitary- and mouse pancreas-derived G protein-coupled receptor proteins. The polypeptide has central nervous system function modulating action but no mentioning of effect in pain treatment is mentioned.

### SUMMARY OF THE INVENTION

The object of this invention is to characterize the sites of expression of the RF-amide peptide PrRP, and the corresponding gene expression in the central and peripheral nervous systems to identify the sites where application of the peptide or related receptor ligands will modify acute or chronic pain sensation.

Another object of this invention is to provide potential applications of the PrRP peptide, its C-terminal peptide fragment and the corresponding receptor in developing treatments to disorders of the brain and central nervous system.

In the invention it was found out that PrRP is expressed in areas important in autonomic regulation and pain, and that PrRP and its C-terminal fragments display potent effects useful in drug development.

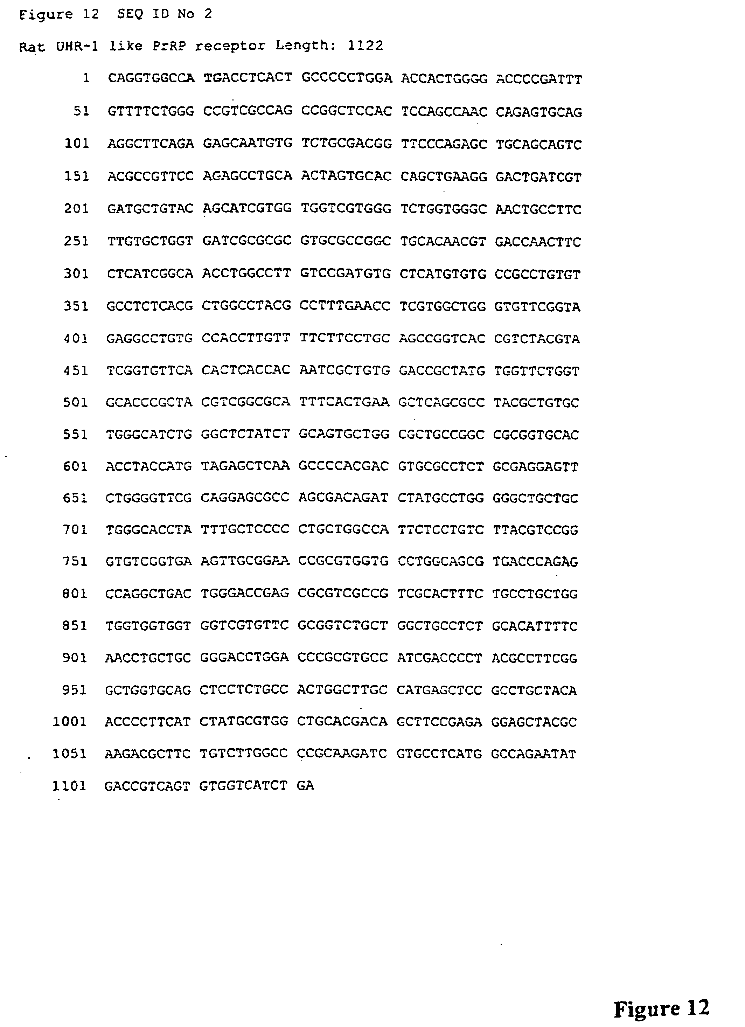

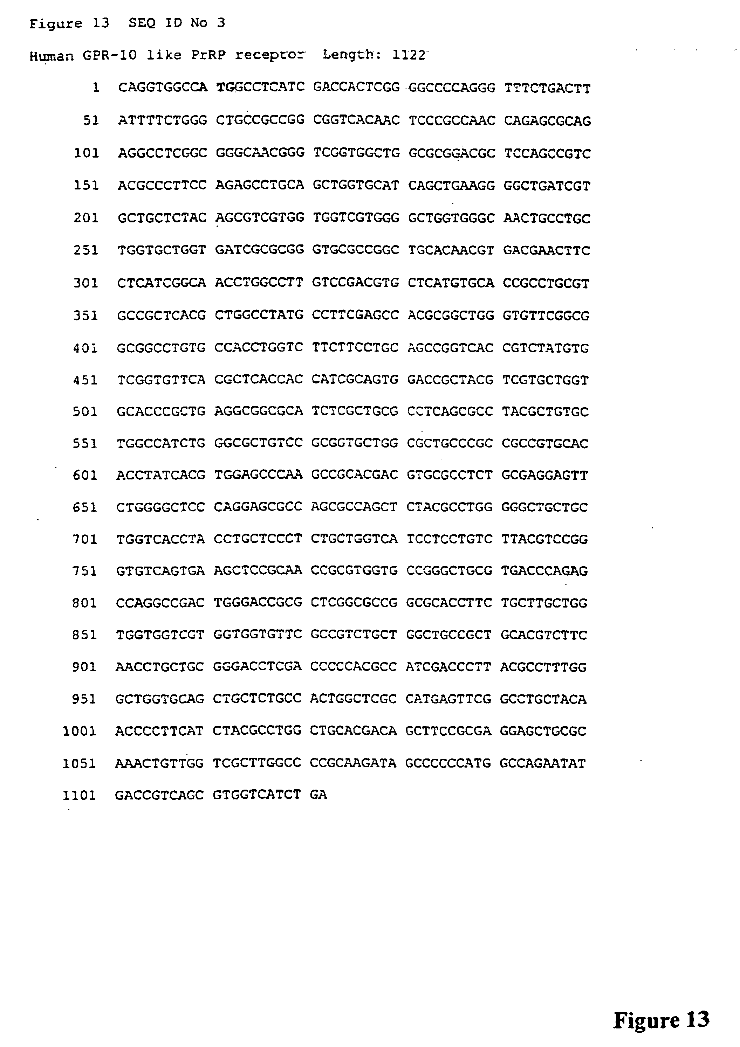

The PCR cloned PrRP receptor (a G-protein coupled receptor resembling GPR10 and UHR1, previously known as an orphan receptors) was expressed in area postrema, an area relevant for regulation of blood pressure and other autonomic functions. Both the human and the rat receptor were cloned. The receptor sequences are presented in figures 12 and 13.

### BRIEF DESCRIPTION OF THE FIGURES

The invention will now be described in detail with reference to the figures, wherein:

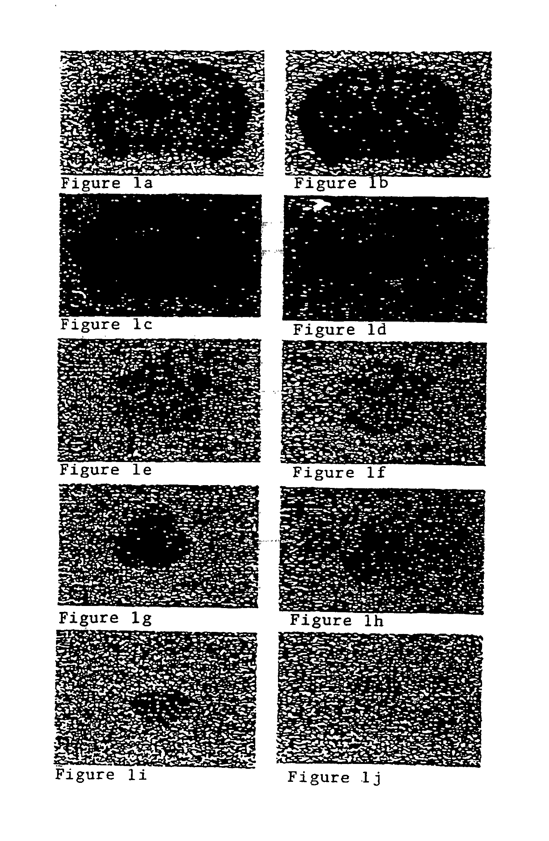

FIG. 1 shows PrRP gene expression in the rat CNS relative to the other RF-amide peptide gene, NPFF using in situ hybridization. The left side shows PrRP, right side NPFF expression. A) PrRP mRNA is absent from the supraoptic and paraventricular nucleus, B) NPFF is expressed in the supraoptic and paraventricular nuclei. C) PrRP mRNA expression is seen in the dorsomedial nucleus of the hypothalamus, D) NPFF mRNA is absent from the same area, E) No PrRP mRNA expression is evident in the rostral nucleus of the solitary tract (NTS), F) Strong expression of NPFF mRNA is seen in the same area, G) The caudal commissural part of the NTS displays strong PrRP mRNA signal, H) The dorsal spinal cord is devoid of PrRP mRNA, I) No PrRP mRNA expression is seen in the dorsal horn of the spinal cord, J) Strong NPFF mRNA expression is seen in the same site.

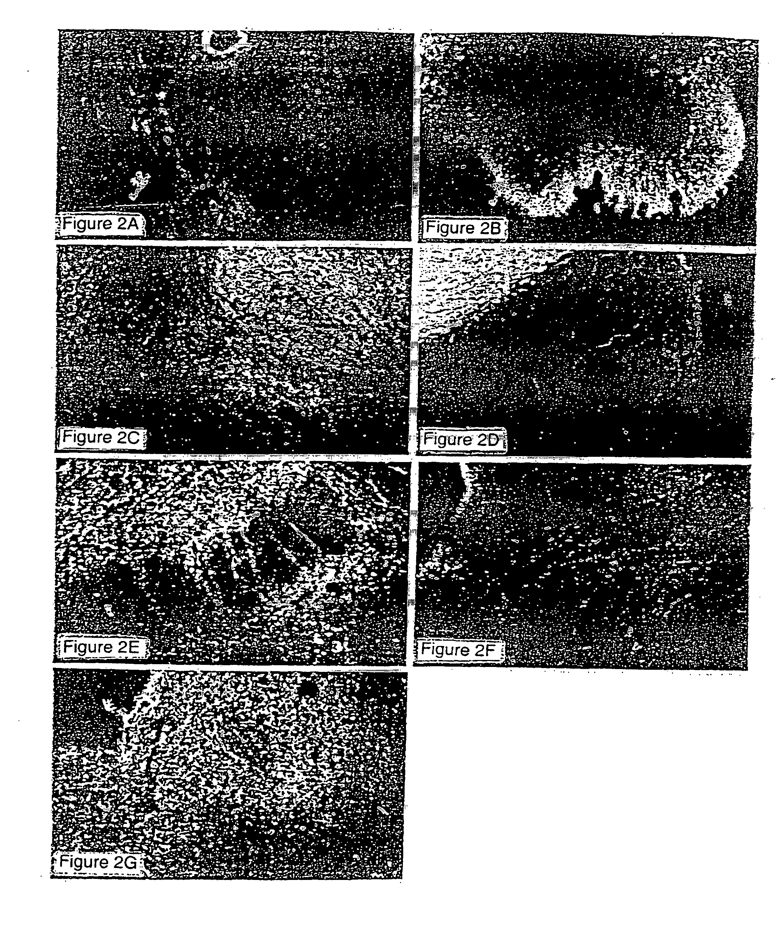

FIG. 2 is a representation of immunohistochemistry of PrRP in rat brain using C-and N-terminal antisera:
   A) Nerve cell bodies in the commissural part of the NTS displaying immunoreactivity for the N-terminus of PrRP20. Dorsal is to the left
   B) Strong PrRP immunoreactivity in the median eminence using an antiserum against the C-terminal peptide PrRP8
   C) Nerve fibers in the bed nucleus of the stria medullaris display immunoreactivity for PrRP
   D) Nerve fibers immunoreactive for PrRP in lateral septum
   E) Nerve fibers immunoreactive for PrRP in the lateral and medial parabrachial nucleus
   F) Nerve fibers immunoreactive for PrRP in the rostral part of the nucleus of the solitary tract
   G) Nerve fibers immunoreactive for PrRP in the ventrolateral periaqueductal grey.

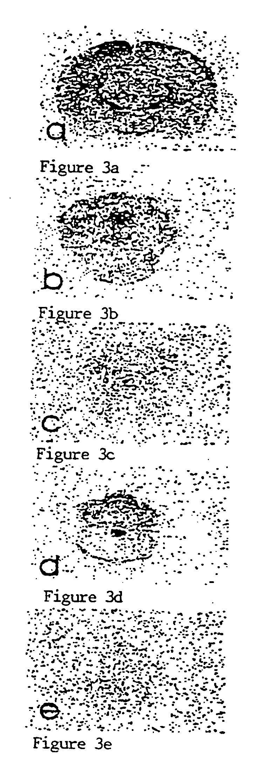

FIG. 3 shows expression of the PrRP receptor mRNA in the brain. A) A strong receptor expression is seen in the reticular nucleus of the thalamus and associated with the periventricular zone of the third ventricle in the hypothalamus. B) PrRP receptor mRNA in the dorsal pons/medulla, C) a control probe shows no signal in a corresponding section, D) Area postrema displays strong receptor signal, E) a control probe shows no signal in a corresponding section.

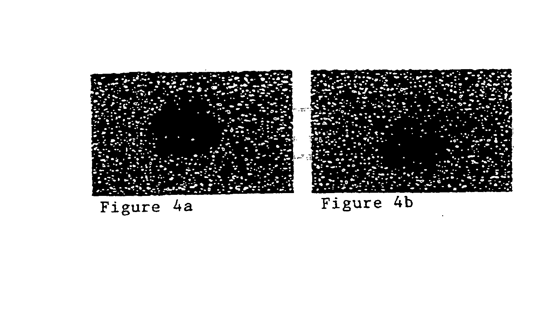

FIG. 4 shows the specificity of the PrRP in situ hybridization in two consecutive sections. A) Strong signal is seen when the section is hybridized with an antisense probe, B) No signal is evident when the section is hybridized with a sense probe.

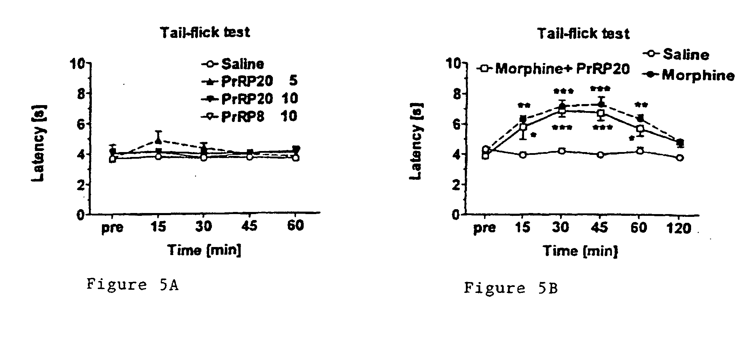

FIG. 5 is a curve showing the effect of intrathecally adminstered PrRP20 and PrRP8 on acute pain
   A) Effect of PrRP20 (at doses 5 and 10 nmol) or its C-terminal octapeptide PrRP8 (at dose 10 nmol) on tail-flick latency time in normal rats
   B) Effect of morphine and morphine + PrRP20 (5 nmol) on tail-flick latency time in normal rats.

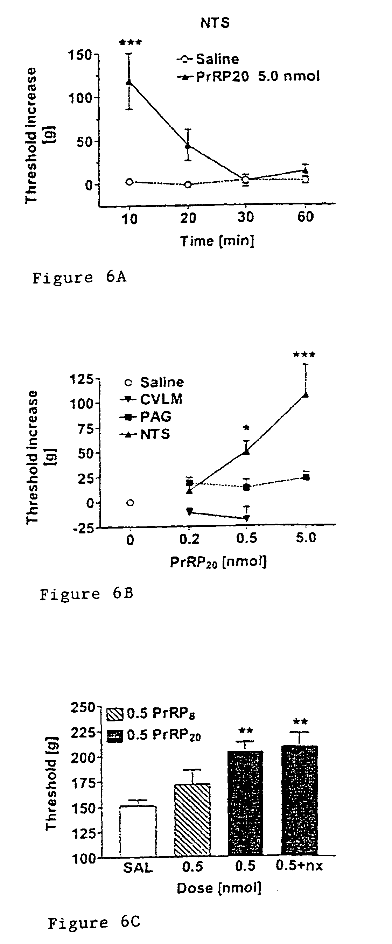

FIG. 6. Hindlimb withdrawal threshold induced by noxious mechanical stimulation (paw pressure test) following PrRP20 or saline in the brainstem. A) PrRP20 (5 nmol) produced a significant increase in the threshold 10 min following the injection in the NTS. The threshold was at control level within 30 min. Saline did not induce any change in the threshold. *** p<0.005 (Tukey's test; ref: the corresponding threshold prior to drug injection). B) The dose-dependence of threshold elevation 10 min following injection of PrRP20 in the NTS, CVLM (caudal ventrolateral medulla) or PAG (periaqueductal grey). Saline had no effects, whereas PrRP20 in the NTS produced a dose-dependent threshold elevation. The increase of threshold by PrRP20 in the PAG was short of significance, and also the decrease of threshold by PrRP20 in the CVLM was short of significance. * p<0.05, *** p<0.005 (Tukey's test; ref: the saline group). C) Threshold 10 min following microinjection of saline, PrRP8 (0.5 nmol), PrRP20 (0.5 nmol) or following PrRP20 (0.5 nmol) together with naloxone (1 mg/kg s.c. 5 min prior to PrRP20 injection). All microinjections into the NTS. PrRP20 produced a significant threshold elevation that was not reversed by naloxone. The effect of PrRP8 was short of significance. In each group n=4-10.

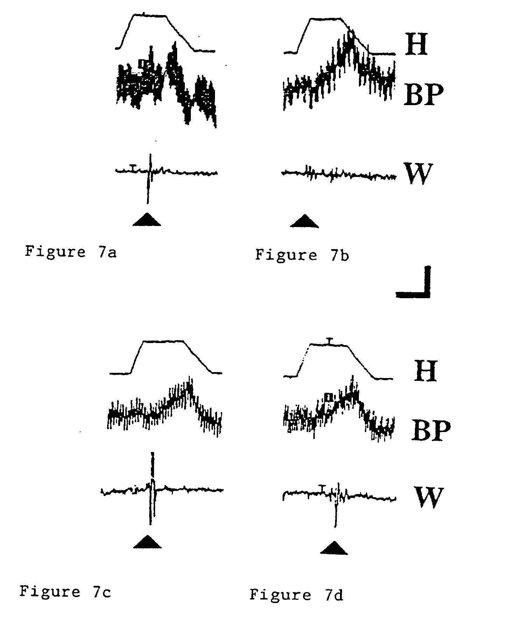

FIG. 7. An example of heat evoked-blood pressure increase and hindlimb withdrawal latency following microinjection of PrRP20 (0.5 nmol) in the CVLM vs. NTS. A) Prior to injection into the CVLM. B) Five min following injection into the CVLM. C) Prior to injection into the NTS. D) Five min following injection into the NTS. Note that the heat-evoked blood pressure increase is higher following PrRP20 in the CVLM and this is accompanied by a decrease in latency of hindlimb withdrawal.
   H indicates the heat stimulus (from the baseline of 35 °C to peak temperature of 54 °C). BP indicates the blood pressure. W indicates the hindlimb withdrawal measured with a piezoelectric device (arrow indicates the start of the withdrawal). The horizontal calibration bar represents 5 s, and the vertical one 20 mmHg for BP curves.
FIG. 8. A) The latency of the heat-evoked hindlimb withdrawal following microinjection of PrRP20 (0.5 nmol) or saline in the NTS or CVLM. B) The change in baseline (mean arterial) blood pressure 5 and 15 min following injection of PrRP20 in the NTS or CVLM. C) The heat-evoked increase in blood pressure following PrRP20 in the NTS or CVLM. In B and C, 0 represents the corresponding pre-drug value. *p<0.05 (Tukey's test; ref: the saline group). n=4 in each group.
FIG. 9. The antiallodynic effect of PrRP20 in the NTS and PAG in neuropathic rats. A) PrRP20 in the NTS produced a dose-dependend attenuation of tactile allodynia in neuropathic animals. According to Friedman ANOVA, the antiallodynia was significant at doses 0.5 and 5 nmol, but not at the dose of 0.2 nmol. B) PrRP20 in the PAG also produced a significant attenuation of tactile allodynia at the dose of 0.5 nmol but not at the dose of 0.2 nmol. n=4-6 in each group.
Fig. 10 shows the effect of intravenously adminstered PrRP8 on blood pressure.
   A) Effect of intravenous PrRP8 (18 µg/kg) on blood pressure in the rat
   B) Effect of intravenous PrRP8 (45 µg/kg) on blood pressure
   C) Effect of intravenous PrRP8 (90 µg/kg) on blood pressure
   D) Effect of intravenous PrRP8 (126 µg/kg) on blood pressure.
FIG. 11. Effect of intravenously injected PrRP20 on blood pressure.
   A) Saline injection did not affect blood pressure in the rat
   B) PrRP20 at a dose of 220 µg/kg induced a rapid increase in blood pressure in the same rat.
Fig. 12 shows the rat receptor sequence SEQ ID NO:2.
Fig. 13 shows the human receptor sequence SEQ ID NO:3.

### DETAILED DESCRIPTION OF THE INVENTION

The experiments carried out and described here were performed in rat and mouse. The presence and expression of the PrRP peptide and its corresponding receptor are believed to be present in similar locations and display similar functions in other mammals including man.

The presented invention is directed to the observation that PrRP is a central regulator of pain mechanisms and autonomic functions. The expression pattern of the peptide with nerve cell bodies expressing both mRNA and peptide immunoreactivity in NTS (nucleus tractus solitarii) and ventrolateral medulla indicate that the PrRP peptide is found in appropriate neural systems. Furthermore, the nerve terminals displaying PrRP-immunoreactivity were found in these areas, and in the parabrachial area and the periaqueductal grey, which indicates that these areas not only contain cells capable of producing the peptide but are also targets of their innervation. The PrRP receptor was also found in an area suitable for such a function. Finally, direct application of the peptide into these areas induced strong analgesic (NTS) and mild hyperalgesic (CVLM) effects, indicating directly the site (NTS) in the brain where the analgesia is generated by PrRP20. Furthermore, PrRP20 had an antiallodynic effect on neuropathic animals when adminstered into the NTS or PAG. In addition, recordings of blood pressure indicated that PrRP20 in the CVLM facilitates heat-evoked blood pressure response (a somato-autonomic reflex) concomitantly with the facilitation of heat-evoked hindlimb withdrawal response (a somatomotor reflex). This finding indicates that PrRP20 in the CVLM has pain facilitatory effects.

Different effects indicate that several neural systems within the medulla are involved, and suggest that the effects may be modulated by intemeurons. Lack of analgesic effects in the spinal cord is in agreement with lack of peptide immunoreactivity and lack of PrRP mRNA. It is also a clear indication that the mechanism that PrRP utilizes differs from those of NPFF.

The nucleus of the solitary tract and ventrolateral medulla are important for regulation of blood pressure. Strong cellular and terminal PrRP-immunoreactivity in these locations suggests that blood pressure may be regulated by PrRP, and direct intravenous injection of the peptide showed this to be true. However, strong expression of the PrRP receptor in the area postrema, an area immediately adjacent to nts and involved in autonomic regulation, suggests that also bloodborne peptide may potentially modulate autonomic functions through this site. It seems that the receptor protein is produced in area postrema, transported to distal ramifications, which extend to e.g. NTS, one candidate structure for mediation of effects on blood pressure.

Here we find that the RF-amide peptide genes (PrRP and NPFF) are expressed in restricted areas of the CNS only, and their functions are therefore likely to be highly specific. It is shown for the first time that the mammalian RF-amide peptide gene PrRP is highly expressed in the medulla and moderately expressed in the hypothalamus, whereas almost all other brain areas display very low or no expression at all, and nerve terminals containing the active peptide display limited distribution in areas relevant for regulation of pain, autonomic functions, hormonal regulation and limbic functions. More specifically, the peptide and its receptor appear to be important in regulation of autonomic functions mediated by medullary mechanisms. These functions include blood pressure and heart regulation. On the other hand, the peptide is found in the hypothalamus, where it is involved in hormonal regulation. Considering the unique projections of RF-amide related peptide systems from the hypothalamus to both limbic areas (amygdala) and medulla (Aamisalo et al. 1995, Panula et al. 1996) and the expression of PrRP gene in this nucleus as shown here, this RF-amide containing peptide PrRP may also play essential roles in limbic functions including emotional components of feeding and drinking, and memory. Intrestingly, the expression of the RF-amide peptide genes was restricted to only a few sites in the CNS, and these were complementary rather that overlapping: NPFF was expressed strongly in the dorsal horn of the spinal cored, whereas PrRP was absent form this site. In the medulla, PrRP was found in the caudal and commissural parts of the nts, whereas NPFF was limited to the rostral parts of the nts. In the hypothalamus, PrRP was not found in the supraoptic and paraventricular nuclei, which contained NPFF mRNA expressing neurons. The area between the dorsomedial and ventromedial nucleus harbored a group on neurons, which displayed PrRP but not NPFF mRNA.

The fact that the expression levels of NPFF and PrRP genes in the periphery appears to be very low (data not shown) suggests that it may be possible to plan treatment of CNS disorders, especially those related to autonomic functions and hormonal regulation, without major peripheral side effects.

The main site of PrRP gene expression in rat medulla oblongata, the commissural nucleus tractus solitarii receives a major input from glossopharyngeal and vagus nerves, and signals to the forebrain, mesencephalon and pons, especially to cells that control the autonomic and neuroendocrine functions. It is also reciprocally connected to groups of cells, which innervate sympathetic and vagal preganglionic neurons, thus creating a putative feedback mechanism (Loewi and Spyer 1990).

Sympathetic outflow is regulated by reciprocal pathways between the nts, ventrolateral medulla, the catecholaminergic cell groups, and the parabrachial nucleus (Loewi and Spyer 1990). All of these areas contain components of the PrRP neuronal system, which suggests that the peptide may regulate sympathetic functions by several mechanisms.

This is the first report on the effect of PrRP on pain in normal and neuropathic animals. It is particularly interesting that the effect is limited to the brain, and spinal mechanisms do not seem to be involved. Furthermore, application of the peptide to another site (CVLM) had a hyperalgesic effect. The site-specific nature of the effects suggests that multiple mechanisms may operate in the medulla.

Taken together, the results indicate that two RF-amide peptide encoding genes, PrRP and NPFF, are expressed in a complementary manner in the medulla oblongata. Crucial sites for regulation of pain and autonomic functions, including blood pressure, are innervated by nerve fibers immunoreactive for PrRP, and the corresponding receptor is also found in area postrema, an important regulatory site in autonomic functions. Pharmacological treatment of rats confirmed that PrRP and itse C-terminal octapeptide strongly modulate blood pressure, whereas only the full-length PrRP20 modulated pain responses in rats. The results also therefore suggest that the C-terminal octapeptide is sufficient for some pharmacological actions, whereas longer fragments are needed for other functions. Also a shorter C-terminal sequences of PrRP are likely to be active.

It was found that administering the PrRP20 peptide or its C-terminal fragment PrRP8 intravenously to an animal increases the arterial blood pressure. Increase in blood pressure is important for example in schock treatment and also in some other conditions where blood pressure is decreasing. This indicates also that blockage of PrRP C-terminal receptors in the central nervous system, especially in the medulla oblongata, and periphery is a useful mechanism in treatment of high blood pressure.

In the performed experiments it was found out that intrathecal PrRP20 peptide did not modulate acute pain, neuropathic pain or morphine analgesia, which is in agreement with the lack of peptide gene expression and immunoreactivity in the spinal cord. However, when administered directly into the nucleus of the solitary tract, the peptide had a potent analgesic effect in the paw pressure test. In the ventrolateral medulla, the peptide displayed a mild hyperalgesic effect. In the central grey the peptide was noneffective except in neuropathic animals. The results suggest that PrRP20 displays site-specific effects on analgesia, which are not mimicked by the shorter C-terminal peptide PrRP8.

It is also evident that a person suffering from a disorder regulated by a receptor located in the central nervous system can be treated by administering to said person an antagonist or agonist to the PrRP receptor. Agonists and antagonists of PrRP20 peptide acting through the corresponding receptor are useful in treatment of acute pain, inflammatory pain and neuropathic pain.

Therapeutic compositions may be provided comprising the PrRP peptide or its C-terminal fragments with a pharmaceutically acceptable carrier or diluent.

Administering of PrRP or its C-terminal octapeptide to rats has been performed intravenously with doses of 18-220 µg/kg for regulating blood pressure, and intrathecally with doses 0.5-10 nmol for treating pain. For humans the doses are probably smaller per weight.

In addition to autonomic and sensory regulation, gene expression data suggests that PrRP may be involved in a number of other functions. It has been previously found that the central hypothalamic area between the dorsomedial and ventromedial nuclei contains cells immunoreactive for RF-amide peptides (Kivipelto et al. 1989, 1991). These cells send projections to both limbic and medullary areas, including amygdala and nts (Aamisalo and Panula 1995). These cells are thus critically positioned in the hypothalamus to link the medullary autonomic regulatory centers to the limbic system (Panula et al. 1996). It is likely that PrRP, or a closely related peptide, would play crucial roles in regulating the limbic functions.

Expression of the PrRP receptor in the thalamic reticulary nucleus suggests that a PrRP-like peptide may regulate cortical information gating by modulating thalamic input to cortex. This would indicate that RF-amide peptides including PrRP, although not prominent in cortical areas, may participate in regulation of higher functions including attention, alertness and memory, and epilepsy, through thalamic and limbic systems.

The invention also suggests that it can be provided a method of activation of RF-amide receptors in the lactotrope cells to induce prolactin release and thus to develop receptor-specific ligands using binding analysis of RF-amide peptides on membranes of these cells, or prolactin release as a test assay.

It is also possible to provide diagnostic methods for identification of genetic disorders involving altered nervous and hormonal function. Diagnostic procedures for hormonal and CNS disorders, including those associated with female reproduction and lactation, pain or autonomic regulation, or male fertility can be based on the sequence of the coding area or regulatory domain of the PrRP gene. These may utilize the specific antisera developed here against the N-and C-terminal domains of PrRP20.

The invention will be further described with reference to the following non-limiting examples.

### Example 1.

### Cloning of a GPR10/UHR-1-related PrRP receptor

The following primers were used to clone a receptor closely resembling the one reported to respond to PrRP in biological assays:

The primers were designed from the published sequence of UHR-1 (Welch et al., 1995). The cloned rat receptor (SEQ ID No 2) had 4 base substitutions resulting in 3 amino acid differences as compared to the previously reported receptor (Welch et al., 1995) and it differed from the reported PrRP receptor (Hinuma et al. 1998). The corresponding human sequence was also revealed (SEQ ID No 3). The cloned human receptor had a one-base difference with the sequence described by Hinuma et al., resulting in a difference in one amino acid (Hinuma et al., 1998). The sequences are shown in Figures 12 and 13.

For cDNA synthesis RNA was isolated from lactating rat brain by RNazol B (Tel Test, Inc., TX, USA). Synthesis of the complementary DNA was performed with M-MuLV reverse transcriptase (Pharmacia). This cDNA was used as template in PCR amplification by Dynazyme II polymerase (Finzymes) with the above mentioned primers:
95 °C for 1 min, 55 °C for 30 s, 72 °C for 30 s and repeated for total of 30 cycles.

The program was run across a temperature gradient (T= 58 °C +/- 10 °C) with Mastercycler Gradient Machine (Eppendorf).

### Example 2.

### Generation of antibodies to PrRP

The peptides TPDINPAWYAGRGIRPVGRF-NH2 (corresponding to PrRP20) (SEQ ID NO:1) and the C-terminal fragment GIRPVGRF-NH2 (corresponding to PrRP8) were synthesized using the solid-phase system, and purified by serial reversed-phase HPLC runs. The purity was confirmed from a single HPLC peak using mass spectrometric analysis. They were coupled to succinylated keyhole limpet hemocyanin (KLH; Sigma, St. Louis, MO) with 1-ethyl-3,3 (dimethylaminopropyl) carbodiimide (EDAC; Sigma) as described earlier (Panula et al. 1982) and antisera were produced in rabbits.

The same peptides were also synthesized as multiple-antigenic peptides (MAPs) to facilitate antibody production. The MAPs were injected intradermally to rabbits with 500 µg of the conjugate of MAP peptide with 500 µl of Freund's complete adjuvant. After five weeks, a booster injection was given (300 µg of conjugate in 500 µl of Freund's incomplete adjuvant). Sera were collected and tested for presence of antibodies against PrRP-like peptides using dot-blot tests and immunocytochemistry using brain sections through areas containing PrRP mRNA. Specific antisera, which recognized neural structures in the brain and gave no such reaction when preadsorpbed with corresponding peptides, were generated.

### Example 3.

### In situ hybridization probes

Oligonucleotides for *in situ* hybridization were generated with a DNA synthesizer according to the published sequences of rat NPFF, PrRP and PrRP-receptor. Probes NPFF (CAA GCA TTT CTA CCA AAC CTC TGG GGC TGA AAC AAG AAG GCT GGG TTC CTT CTA and GGG AAG TGA TTT TGC ATG CAG ACA TAT CAC AGC AGA TGA TGT TAC TTC TT), PrRP (TTG ATA CAG GGG TTC TTGG TCT CCA TGG AGT GCT GGT GGG CTC GGCC CTG GA), GRP 10/UHR-1-related PrRP receptor (TGC GC TCT GGG AAC CGT CGC AGA CAC ATT GCT CTC TGA AGC CTC TGC ACT and AGC GCC AGC ACT GCA GAT AGA GCC CAG ATG CCC AGC ACA GCG TAG GCG CTG) were labelled with deoxyadenosine 5'-α(-thio)triphosphate (³⁵S) (NEG-034H) (DuPont NEN Research products, Boston, MA, USA) at their 3'-ends using terminal deoxynucleotide transferase (Promega, Madison, WI, USA) according to the manufacturer's protocol. The purifications were done in Sephadex G-50-columns. The labelled probes with specific activities of 1-2 x 10⁹ cpm/µg were stored at - 20 °C in 10 mM dithiotreitol (DTT) until used. A 50-mer oligonucleotide probe complementary to Staphylococcus Aureus chloramphenicol acetyltransferase was used as one of the controls.

### Example 4.

### In situ hybridization

The procedure used for *in situ* hybridization with oligonucleotide probes has been described before (Dagerlind *et al*., 1992) and was used with minor modifications. Before hybridization with oligonucleotide probes, the sections were left to air-dry at room temperature for 30 min. The slides were illuminated with UV light for 5 min at a distance of 25 cm to increase the hybridization (Schambra *et al*., 1994). Sections were hybridized 20-24 h at 45 °C (PrRP), 50 °C (PrRP-receptor) or 55 °C (NPFF) in a humidified chamber with 200 µl of hybridization buffer containing 50% deionized formamide, 4 x SSC (0.6 M sodium chloride, 0.06 M sodium citrate), 1 x Denhardt's solution (0.02% polyvinylpyrrolidone, 0.02% Ficoll, 0.02% bovine serum albumin), 1% sarcosyl (N-lauroylsarcosine), 0.02 M sodium phosphate (PB; pH 7.0), 10% dextran sulphate, 500 µg/ml denaturated salmon sperm DNA (ssDNA), 250 µg/ml transfer RNA (tRNA), 200 mM DTT and 10⁷ cpm/ml of the labeled probe (NPFF, PrRP or PrRP-receptor) or *S*. *aureus* chloramphenicol acetyltransferase control probe. After hybridization the slides were dipped in 1 x SSC at room temperature, shortly washed with 1 x SSC at 56 °C and then three times 20 min at 56 °C in 1 x SSC. They were left to cool to room temperature in fresh 56 °C 1 x SSC before dehydration with ethanol (a dipp in distilled water and then 30 sec each in 60%, 80% and absolute ethanol). Tissue sections were then apposed to Kodak BioMax MR-film for 10 days and after that dipped in Kodak NTB2-emulsion and exposed for 50 days. As an additional control, along with the *S. aureus* chloramphenicol acetyltransferase probe, a competitive hybridization was performed with a 100-fold excess of unlabelled NPFF, PrRP or PrRP-receptor oligonucleotide in the hybridization buffer, to abolish all specific hybridization signal. Representative slides from all brain areas were stained with toluidine blue or cresyl violet to allow identification of brain nuclei, neurons.

The two genes encoding for RF-amide peptide genes (NPFF and PrRP) were expressed in key areas of the brain known to be involved in regulation of blood pressure and heart functions, and hormonal regulation. Specifically PrRP was expressed in dorsal medulla oblongata, including the nucleus of the solitary tract and ventrolateral medulla, and in central hypothalamus. NPFF was expressed in the solitary tract nucleus, in the spinal trigeminal nucleus and in the supraoptical and paraventricular nucleus of the hypothalamus. NPFF but not PrRP was expressed in the dorsal horn of the spinal cord.

The receptor for RF-amide peptides related to GPR10 was expressed in central hypothalamus, thalamic reticular nucleus and medulla oblongata, in particular in the area of postrema.

### Example 5.

### Image analysis

Quantification of autoradiographic *in situ* hybridization films was done by digitizing the film images with a computer-based MCID image analysis system (Imaging Research, St Catherines, Ontario, Canada) and by measuring different brain areas in gray-scale pixel values. The relative optical density is converted to a linear gray scale value based on an appropriately derived ¹⁴C-standard curve essentially as described in our previous reports (Lintunen et al. 1998, Vilim et al. 1999).

### Example 6.

### Immunocytochemistry

Immunohistochemistry was performed using specific antisera against the C-termini of the two active peptides, NPFF and NPSF, present in the NPFF precursor, and PrRP. Normal or colchicine-treated male Wistar rats (250-300 g) were perfused with 4% paraformaldehyde and brain sections were processed for immunofluorescence as described (Kivipelto et al., 1989). Primary antisera were diluted 1:500 - 1:20 000, and preadsorption controls with several peptides were carried out as described earlier (Kivipelto et al.,1989).

For comparison, antisera against the NPFF gene-related peptides NPFF and NPSF were similarly applied. Data for PrRP is illustrated in FIG. 2.

Immunocytochemistry with antibodies against NPSF, a typical RF-amide peptide, revealed extensive fiber and terminal networks in the lateral the parabrachial nucleus, the solitary tract nucleus, ventrolateral medulla, amygdala, bed nucleus of the stria medullaris, and several hypothalamic nuclei. The antiserum was C-terminally specific, which suggests that the immunolocalization represents a group of related peptides. Immunocytochemistry of PrRP verified the results obtained for corresponding mRNA with in situ hybridization and confirmed that PrRP gene-derived peptides are formed and stored in key regulatory areas of the medulla. Large neurons displaying strong TPDINPAWYAGRGIRPVGRF-NH2-immanoreactivity (PrRP20-ir) were seen in the commissural part of the nucleus of the solitary tract. Fiber projections emanating from this area were seen to extend to anterior and posterior directions, and to the ventrolateral direction. Extensive terminal networks were seen in other parts of the nucleus of the solitary tract, trigeminal complex and several nuclei of the ventral and lateral medulla oblongata, lateral and medial parabrachial nucleus and ventrolateral periaqueductal grey. Nerve cell bodies were also seen in the ventrolateral medulla and the hypothalamus on the level of dorsomedial and ventromedial nuclei. Fibers were also seen in the several hypothalamic sites, most notably in the median eminence with the c-terminally oriented antiserum, and in the paraventricular and periventricular areas and lateral hypothalamus with both c-terminally and N-terminally oriented antiserum. Of the limbic areas, the bed nucleus of the stria terminalis and lateral septum displayed distinct fiber networks. Dense fiber networks in the paraventricular and lateral hypothalamic areas indicate involvement of the PrRP in regulation of food intake.

### Example 7.

### Neuropathic pain model

The model of Kim and Chung was applied as described earlier (Kontinen et al. 1997) was used. The animals were anesthetized with halothane, and left L5 and L6 spinal nerves were exposed, isolated and ligated tightly with 6-0 silk thread. Rats, which developed significant mechanical allodynia (threshold for paw withdrawal after von Frey hair stimulation with the force of 4.2 g or less) at two weeks from the ligation were used. Intrathecal cannulation was done as described (Kontinen et al. 1997).

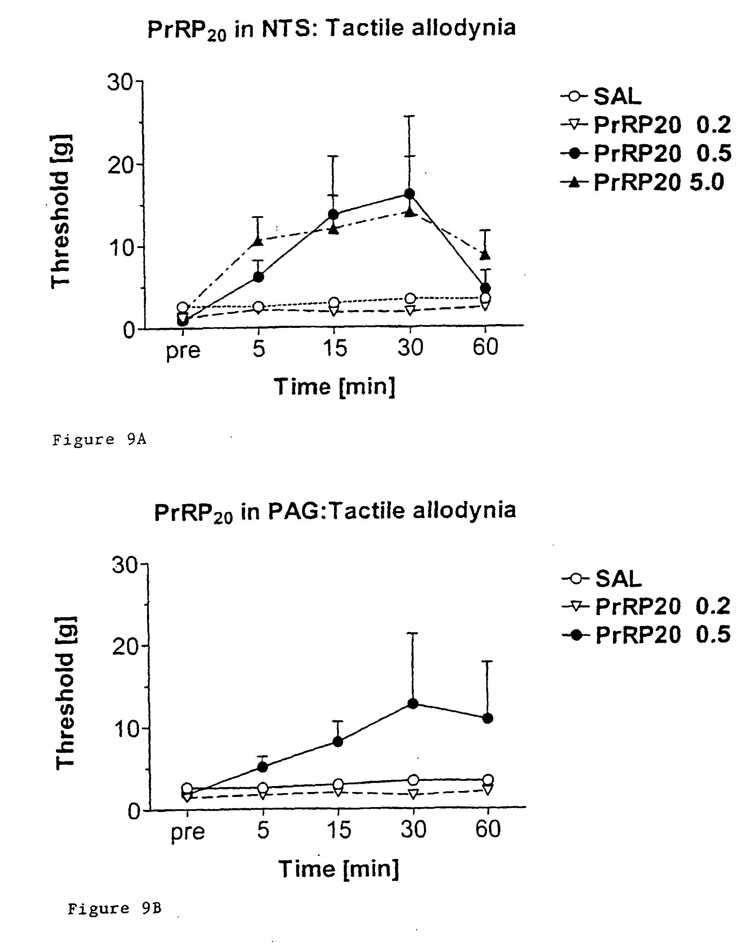

The results of intrathecal injections are presented in figure 5. From the figure it can be seen that intrathecal PrRP had no effect on pain in this model. PrRP20 in the NTS and also in the PAG had a significant antiallodynic effect in neuropathic animals (Fig. 9).

### Example 8.

### Testing of analgesia and allodynia

The heat-induced tail flick response was determined using a radiant heat device. The heat beam was focused on the tail and the tail flick time was recorded.

Intrathecal adminstration of PrRP revealed that the peptides were ineffective on the level of the spinal cord. In normal rats, there were no significant antinociceptive effects (tail flick test) with either PrRP or its C-terminal octapeptide fragment at doses 0.5, 5 or 10 nmol (Fig. 5A). An i.t. dose of 5 nmol did not significantly modify the antinociceptive effect of morphine (Fig. 5B). In neuropathic rats, i.t. doses of 5 or 10 nmol of the C-terminal octapeptide did not change cold allodynia or mechanical allodynia. Injection of the PrRP20 peptide into the NTS induced a strong antinociception after mechanical stimulation. The effect was evident 5 min after admistration, maximal (20 min (Fig. 6). PrRP8 was ineffective at NTS (Fig. 6). In the ventrolateral medulla (CVLM), similar injection of PrRP20 induced mild hyperalgesia (Fig. 6B). In the periaqueductal grey the peptide was ineffective when tested in healthy animals (Fig. 6B). The antinociceptive effect induced by intracerebral drug injections was tested in healthy animals using paw pressure test as described earlier (Wei et al., 1998). The technique of intracerebral drug injections is described in detail elsewhere (Wei et al. 1998). Testing of tactile allodynia was performed using a series of calibrated monofilaments as described earlier (Wei et al., 1998).

Adminstration of the peptide into the central grey of neuropathic rats had an antiallodynic effect (Fig. 9B). Also in the NTS PrRP20 had a dose-dependent antiallodynic effect in neuropathic animals (Fig. 9A).

### Example 9.

### Blood pressure recordings after intracerebral injections

Rats were anesthetized with pentobarbitone and placed in a standard stereotaxic frame. For microinjections of the drug into the CVLM or PAG a guide cannula was placed in the brainstem. For recording of hindlimb withdrawal, a piezoelectric device was glued to the hindlimb. For recording of average blood pressure, an intraarterial catheter was placed into carotid artery and it was connected to a calibrated blood pressure transducer (Harvard Apparatus Inc). The noxious heat stimuli (52°C of 5 s duration) were applied from a feedback-controlled contact thermostimulator (LTS3, Thermal Devices Inc,.) to the glabrous skin of the hindpaw. The responses were recorded on a digital storage oscilloscope.

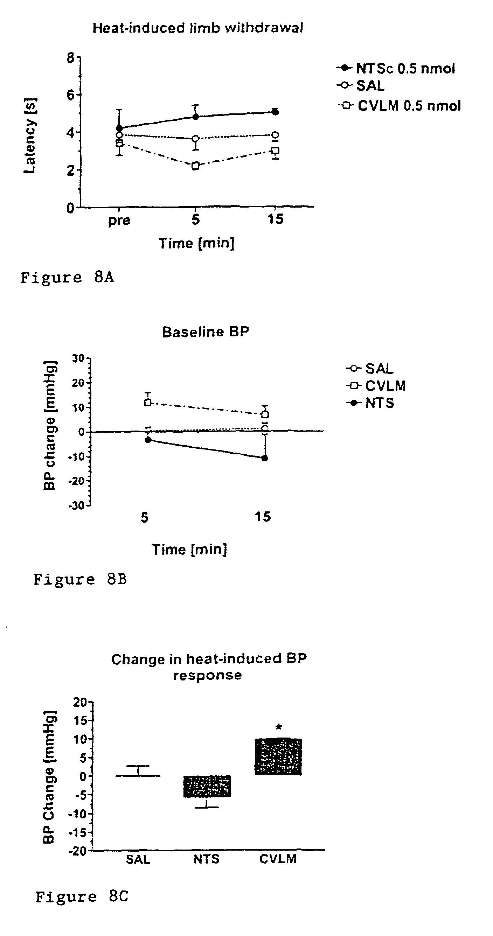

The basal blood pressure, the heat evoked blood pressure increase and the latency of heat-evoked limb withdrawal were recorded prior to drug injections and at various intervals following the drug injections. Recordings of blood pressure indicated that PrRP20 in the CVLM facilitates heat-evoked blood pressure response (a somato-autonomic reflex) concomitantly with the facilitation of heat-evoked hindlimb withdrawal response (a somatomotor reflex). This finding presented in Figs. 7 and 8 indicates that PrRP20 in the CVLM has pain facilitatory effects.

### Example 10.

### Blood pressure measurements after i.v. injections

Rats were anesthetized with urethane or isoflurane and the femoral vein and artery were cannulated. The Grass recording system was used after the pressure gage was connected to the arterial tubing. The peptides TPDINPAWYAGRGIRPVGRF-NH2 (corresponding to PrP20) and the C-terminal fragment GIRPVGRF-NH2 (corresponding to PrRP8) were injected intravenously, and arterial blood pressure was monitored.

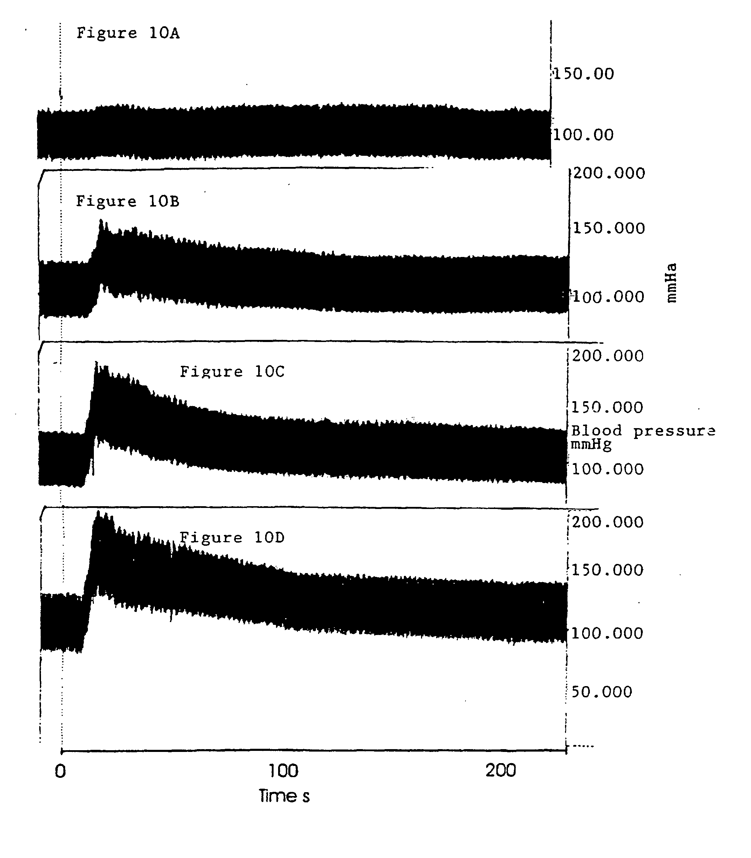

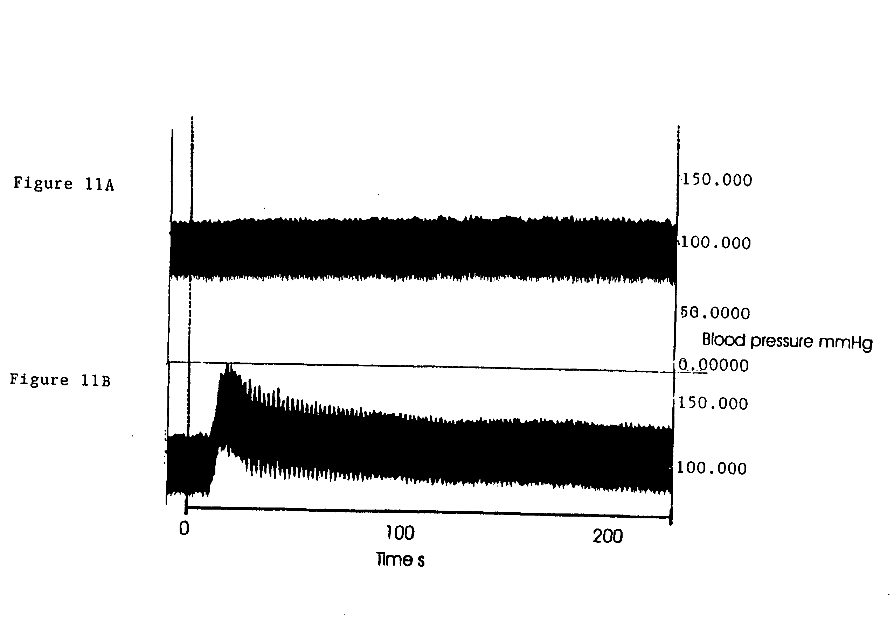

The results are shown in figures 10 and 11. Intravenous injection of PrRP20 at a dose of 220 µg/kg (Fig. 11) or its C-terminal octapeptide fragment PrRP8 at doses 18-126 µg/kg (Fig. 10) induced a clear increase in arterial blood pressure (maximally 70 or 60 mmHg in systolic and 40 and 30 in diastolic pressure, respectively), and increase in heart rate (30-50 bpm, from 360 to 390-420 bpm). In the same animals, saline was without an effect (Fig. 11A) and phenylephrine induced a clear increase in pressure.

### SEQUENCE LISTING

<110> Oy Juvantia Pharma Ltd.
<120> Method for regulating autonomic functions and treating pain
<130> PCT/FI00/00664
<140> PCT/FI00/00664
   <141> 2000-08-03
<150> 09/365756
   <151> 1999-08-03
<150> 09/531567
   <151> 2000-03-20
<160> 3
<170> Patentln Ver. 2.1
<210> 1
   <211> 20
   <212> PRT
   <213> Human
<400> 1
   Thr Pro Asp Ile Asn Pro Ala Trp Tyr Ala Gly Arg Gly Ile Arg Pro
   1 5 10 15
   Val Gly Arg Phe
   20
<210> 2
   <211> 1122
   <212> DNA
   <213> Rat
<400> 2
   caggtggcca tgacctcact gccccctgga accactgggg accccgattt gttttctggg
   60
   ccgtcgccag ccggctccac tccagccaac cagagtgcag aggcttcaga gagcaatgtg
   120
   tctgcgacgg ttcccagagc tgcagcagtc acgccgttcc agagcctgca actagtgcac
   180
   cagctgaagg gactgatcgt gatgctgtac agcatcgtgg tggtcgtggg tctggtgggc 240
   aactgccttc ttgtgctggt gatcgcgcgc gtgcgccggc tgcacaacgt gaccaacttc 300
   ctcatcggca acctggcctt gtccgatgtg ctcatgtgtg ccgcctgtgt gcctctcacg 360
   ctggcctacg cctttgaacc tcgtggctgg gtgttcggta gaggcctgtg ccaccttgtt 420
   ttcttcctgc agccggtcac cgtctacgta tcggtgttca cactcaccac aatcgctgtg 480
   gaccgctatg tggttctggt gcacccgcta cgtcggcgca tttcactgaa gctcagcgcc 540
   tacgctgtgc tgggcatctg ggctctatct gcagtgctgg cgctgccggc cgcggtgcac 600
   acctaccatg tagagctcaa gccccacgac gtgcgcctct gcgaggagtt ctggggttcg 660
   caggagcgcc agcgacagat ctatgcctgg gggctgctgc tgggcaccta tttgctcccc 720
   ctgctggcca ttctcctgtc ttacgtccgg gtgtcggtga agttgcggaa ccgcgtggtg 780
   cctggcagcg tgacccagag ccaggctgac tgggaccgag cgcgtcgccg tcgcactttc 840
   tgcctgctgg tggtggtggt ggtcgtgttc gcggtctgct ggctgcctct gcacattttc 900
   aacctgctgc gggacctgga cccgcgtgcc atcgacccct acgccttcgg gctggtgcag 960
   ctcctctgcc actggcttgc catgagctcc gcctgctaca accccttcat ctatgcgtgg 1020
   ctgcacgaca gcttccgaga ggagctacgc aagacgcttc tgtcttggcc ccgcaagatc 1080
   gtgcctcatg gccagaatat gaccgtcagt gtggtcatct ga 1122
<210> 3
   <211> 1122
   <212> DNA
   <213> Human
<400> 3
   caggtggcca  tggcctcatc  gaccactcgg  ggccccaggg  tttctgactt  attttctggg
   60
   ctgccgccgg  cggtcacaac  tcccgccaac  cagagcgcag  aggcctcggc  gggcaacggg
   120
   tcggtggctg  gcgcggacgc  tccagccgtc  acgcccttcc  agagcctgca  gctggtgcat
   180
   cagctgaagg  ggctgatcgt  gctgctctac  agcgtcgtgg  tggtcgtggg  gctggtgggc
   240
   aactgcctgc  tggtgctggt  gatcgcgcgg  gtgcgccggc  tgcacaacgt  gacgaacttc
   300
   ctcatcggca  acctggcctt  gtccgacgtg  ctcatgtgca  ccgcctgcgt  gccgctcacg
   360
   ctggcctatg  ccttcgagcc  acgcggctgg  gtgttcggcg  gcggcctgtg  ccacctggtc
   420
   ttcttcctgc  agccggtcac  cgtctatgtg  tcggtgttca  cgctcaccac  catcgcagtg
   480
   gaccgctacg  tcgtgctggt  gcacccgctg  aggcggcgca  tctcgctgcg  cctcagcgcc
   540
   tacgctgtgc  tggccatctg  ggcgctgtcc  gcggtgctgg  cgctgcccgc  cgccgtgcac
   600
   acctatcacg  tggagcccaa  gccgcacgac  gtgcgcctct  gcgaggagtt  ctggggctcc
   660
   caggagcgcc  agcgccagct  ctacgcctgg  gggctgctgc  tggtcaccta  cctgctccct
   720
   ctgctggtca  tcctcctgtc  ttacgtccgg  gtgtcagtga  agctccgcaa  ccgcgtggtg
   780
   ccgggctgcg  tgacccagag  ccaggccgac  tgggaccgcg  ctcggcgccg  gcgcaccttc
   840
   tgcttgctgg  tggtggtcgt  ggtggtgttc  gccgtctgct  ggctgccgct  gcacgtcttc
   900
   aacctgctgc gggacctcga cccccacgcc atcgaccctt acgcctttgg gctggtgcag
   960
   ctgctctgcc actggctcgc catgagttcg gcctgctaca accccttcat ctacgcctgg
   1020
   ctgcacgaca gcttccgcga ggagctgcgc aaactgttgg tcgcttggcc ccgcaagata
   1080
   gccccccatg gccagaatat gaccgtcagc gtggtcatct ga
   1122

### References

- Kivipelto,: L., Majane, E.A., Yang, H.-Y.T. and Panula, P.: Immunohistochemical distribution and partial characterization of FLFQPQRFamide-like peptides in the rat central nervous system. J. Comp. Neurol. 286: 269-287, 1989.
- Kivipelto, L.: and Panula, P.: Central neuronal pathways containing FLFQPQRFamide-like (morphine-modulating) peptides in the rat brain. Neuroscience 41: 137-148, 1991.
- Kivipelto,: L. and Panula, P.: Comparative distribution of neurons containing FLFQPQRF-amide-, NPY-and enkephalin-like immunoreactivities in the rat brain. Eur. J. Neurosci. 3: 175-185, 1991.
- Kivipelto,: L. and Panula, P.: Comparative distribution of neurons containing FLFQPQRF-amide-, NPY-and enkephalin-like immunoreactivities in the rat brain. Eur. J. Neurosci. 3: 175-185, 1991.
- Pertovaara,: A., Hämäläinen, M.M., Kauppila, T. and Panula, P.: Carrageenan-induced changes in spinal nociception and its modulation by the brainstem. Neuroreport, 9: 351-355, 1998.
- Wei, H.,: Panula, P., Pertovaara, A.: A differential modulation of allodynia, hyperalgesia and nociception by neuropeptide FF in the periaqueductal gray of neuropathic rats: interactions with morphine and naloxone. Neuroscience, 86: 311-319, 1998.
- Vilim, F.S,: Aamisalo, A.A., Nieminen, M-L., Lintunen, M., Karlstedt, K., Kontinen, V., Kalso, E., States, B., Panula, P. and Ziff, E.: Gene for pain modulatory neuropeptide NPFF: induction in spinal cord by noxious stimuli. Mol. Pharmacol., 55: 804-811, 1999.
- Panula, P.,: Aamisalo, A.A. and Wasowicz, K.: Neuropeptide FF, a new mammalian peptide with multiple functions. Progr. Neurobiol. 48: 461-487, 1996.
- Hinuma S,: Habata Y, Fujii R, Kawamata Y, Hosoya M, Fukusumi S, Kitada C, Masuo Y, Asaano T, Matsumoto H, Sekiguchi M, Kurokawa T, Nishimura O, Onda H, Fujino M (1998) A prolactin-releasing peptide in the brain. Nature 393, 272-276.
- Matsumoto: H, Murakami Y, Horikoshi Y, Noguchi J, Habata Y, Kitada C, Hinuma s, Onda H, Fujino M (1999) Biochemical and Biophysical Research Communications 257, 264-268.
- Welch: SK, O'Hara BF, Kilduff TS, Heller HC (1995) Sequence and tissue distribution of a candidate G-protein coupled receptor cloned from rt hypothalamus. Biochem. Biophys. Res. Comm. 209, 606-613.
- Minami: S, Nakata T, Tokita R, Onodera H, Imaki J (1999) Cellular localization of prolactin-releasing peptide messenger RNA in the rat brain. Neurosci. Lett. 266, 73-75.
- Chen C-T,: Dun SL, Dun NJ, Chang J-K (1999) Prolactin-releasing peptide-immuno- reactivity in A1 and A2 noradrenergic neurons of the rat medulla. Brain Res. 822, 276-279.
- Loewi AD,: Spyer KM (1990) Central regulation of autonomic functions, Oxford Unviersity Press, 390 pp.
- Aarnisalo, A.A.: and Panula, P. (1995): Neuropeptide FF-containing efferent projections from the medial hypothalamus of rat: A Phaseolus vulgaris leucoagglutinin study. Neuroscience 65: 175-192.
- Sundblom,: D.M., Panula, P. and Fyhrqvist, F. (1995): Neuropeptide FF-like immunoreactivity in human plasma. Peptides, 16: 347-350.
- Aarnisalo,: A.A., Nieminen M.-L., Tuominen, R., Panula, P. (1997) Neuropeptide FF as a prolactin releasing hormone. Neuroendocrinol. Lett. 18: 191-196.
- Lintunen,: M., Karlstedt, K., Fukui, H., and Panula, P. (1998) Postnatal expression of the histamine H, receptor gene in the rat brain: correlation to L-histidine decarboxylase expression and local upregulation after limbic seizures. Eur. J. Neurosci., 10: 2287-2301.
- Perry SJ,: Huang E, Huang Y-K, Cronk D, Bagust J, Sharma R, Walker RJ, Wilson S, Burke J (1997) A human gene encoding morphine modulating peptides related to NPFF and FMRFamide. FEBS Lett. 409: 426-430.
- Yang H-YT,: Fratta W, Majane EA, Costa E (1985) Isolation, sequencing, synthesis and pharmacological characterizatin of two brain neuropepitdes that modulate the action of morphine. Proc. Natl. Acad. Sci. USA 82: 7757-7761;
- Kontinen,: V., Aarnisalo, A.A., Idänpään-Heikkilä, J. J., Panula, P. and Kalso, E. (1997) Spinal neuropeptide FF in the rat spinal cord during carrageenan inflammation. Peptides, 18: 287-292.

## Claims

1. Use of a C-terminal fragment of prolactin-releasing peptide (PrRP) having the amino acid sequence of TPDINPAWYAGRGIRPVGRF-NH2 (SEQ ID NO:1) referred to as PrRP20 for preparing a medicament for treating pain.

2. The use of claim 1, wherein said pain is acute pain or neuropathic pain.

## Patentansprüche

1. Verwendung von einem C-Terminal Fragment eines Prolactin auslösenden Peptids (PrRP) das die Amino-säure-Sequenz TPDINPAWYAGRGIRPVGRF-NH2 (SEQ ID No 1) hat, von der die Benennung PrRP20 verwendet wird, als Arzneimittel zur Schmerzenbehandlung.

2. Verwendung nach Anspruch 1, wobei der genannte Schmerz ein akuter Schmerz oder ein neuropathischer Schmerze ist.

## Revendications

1. Utilisation d'un fragment C terminal d'un peptide libérant la prolactine (PrRP) ayant la séquence amino-acide TPDINPAWYAGRGIRPVGRF-NH2 (SEQ ID No 1), appelé PrRP20, pour la préparation d'un médicament pour le traitement de la douleur.

2. Utilisation selon la revendication 1, ladite douleur étant une douleur aiguë ou une douleur neuropathique.
